# EUROPÄISCHE PATENTSCHRIFT

(11) **EP 1 090 029 B1**
(45) Veröffentlichungstag und Bekanntmachung des Hinweises auf die Patenterteilung: **20.03.2002**
(21) Anmeldenummer: 99938196.5
(22) Anmeldetag: 18.06.1999
(51) Int. Cl.: C07J 53/00, A61K 31/565, A61K 31/58

(54) **14,15-CYCLOPROPANOSTEROIDE DER 19-NORANDROSTAN-REIHE, VERFAHREN ZU IHRER HERSTELLUNG UND DIESE VERBINDUNGEN ENTHALTENDE PHARMAZEUTISCHE PRÄPARATE**
14,15-CYCLOPROPANOSTEROIDS OF THE 19-NORANDROSTANE SERIES, METHOD FOR THE PRODUCTION THEREOF AND PHARMACEUTICAL PREPARATIONS CONTAINING SAID COMPOUNDS
14,15-CYCLOPROPANOSTEROIDES DE LA SERIE DES 19-NORANDROSTANES, LEUR PROCEDE DE PRODUCTION ET PREPARATIONS PHARMACEUTIQUES CONTENANT CES COMPOSES

(30) Priorität: 22.06.1998 DE 19827522
(43) Veröffentlichungstag der Anmeldung: 11.04.2001
(73) Patentinhaber: Jenapharm GmbH & Co. KG, 07745 Jena (DE)
(72) Erfinder: SCHWARZ, Sigfrid, D-07743 Jena (DE); SCHUBERT, Gerd, D-07743 Jena (DE); RING, Sven, D-07749 Jena (DE); ELGER, Walter, D-14195 Berlin (DE); SCHNEIDER, Birgitt, D-07745 Jena (DE); KAUFMANN, Günter, D-07743 Jena (DE); SOBEK, Lothar, D-07743 Jena (DE)
(74) Vertreter: Cramer, Eva-Maria
(86) Internationale Anmeldenummer: DE9901795
(87) Internationale Veröffentlichungsnummer: WO9967276

(56) Entgegenhaltungen:
- EP-A- 0 768 316
- DERMAN, RICHARD J.: "Androgens and oral contraception" ANDROG. DISORD. (1995), 301-23. EDITOR(S): REDMOND, GEOFFREY P. PUBLISHER: RAVEN, NEW YORK, N. Y. , XP002118686
- M. OETTEL ET AL: "Das Endokrinologische Profil von Metaboliten des Gestagens Dienogest" PHARMAZIE., Bd. 48, Nr. 7, Juli 1993 (1993-07), Seiten 541-545, XP002118687 VEB VERLAG VOLK UND GESUNDHEIT. BERLIN., DD ISSN: 0031-7144
- M. A. AVERY ET AL: "Synthesis and testing of 17a.beta.-hydroxy-7.alpha.-methyl-D-homoes tra-4,16-dien-3-one: a highly potent orally active androgen" STEROIDS: STRUCTURE, FUNCTION, AND REGULATION., Bd. 55, Nr. 2, Februar 1990 (1990-02), Seiten 59-64, XP002118688 ELSEVIER SCIENCE PUBLISHERS, NEW YORK, NY., US ISSN: 0039-128X
- F. BERTI ET AL: "Androstane Derivative Devoid of Anabolic-Virilizing Effects and Endowed with an Antiglucocorticoid Activity" ARZNEIMITTEL FORSCHUNG. DRUG RESEARCH., Bd. 36, Nr. 9, September 1986 (1986-09), Seiten 1369-1371, XP002118689 EDITIO CANTOR. AULENDORF., DE ISSN: 0004-4172
- F. BERTI ET AL: "Androstane Derivatives - Roxibolone and Decylroxibolone - Devoid of Any Affinity for the Androgenic Prostate and Muscle Receptors" ARZNEIMITTEL FORSCHUNG. DRUG RESEARCH., Bd. 36, Nr. 9, September 1986 (1986-09), Seiten 1372-1374, XP002118690 EDITIO CANTOR. AULENDORF., DE ISSN: 0004-4172

## Beschreibung

Die Erfindung betrifft neue 14,15-Cyclopropanosteroide der 19-Norandrostan-Reihe, deren Herstellung und diese Verbindungen enthaltende pharmazeutische Präparate.

Es werden 14,15-Cyclopropanosteroide der 19-Norandrostan-Reihe der allgemeinen Formel I 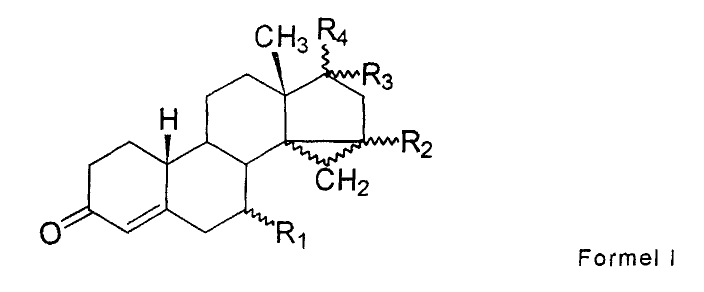 beschrieben.
In der allgemeinen Formel I ist R₁ ein Wasserstoffatom oder ein Alkylrest mit 1-9 Kohlenstoffatomen,
steht R₂ für ein Wasserstoffatom oder eine Methylgruppe,
stehen R₃ und R₄ unabhängig voneinander für ein Wasserstoffatom,
für eine Hydroxygruppe, für eine Acyloxygruppe -O-CO-R₅ mit R₅ für 1-10 Kohlenstoffatome,
für eine Carbamoyloxygruppe O-CO-NHR₆ mit R₆ für ein Wasserstoffatom, einen Alkyl-, Arylrest mit jeweils 1-5 Kohlenstoffatomen,
für eine Sulfamoyloxygruppe -O-SO₂-NR₇R₈ mit R₇ und R₈ unabhängig voneinander jeweils für ein Wasserstoffatom, eine Alkylgruppe mit 1 bis 5 Kohlenstoffatomen oder zusammen mit dem Stickstoffatom für eine Pyrrolidino-. Piperidino- oder Morpholinogruppe,
für eine Gruppierung -CH₂R₉ mit R₉ für eine Hydroxygruppe, eine Alkyloxygruppe mit 1-5 Kohlenstoffatomen, ein Chlor- oder Bromatom, eine Azido-, Nitrilo- oder Thiocyanogruppe oder für eine Gruppierung -SR₁₀ steht mit R₁₀ für eine Alkylgruppe mit 1-5 Kohlenstoffatomen,
oder stehen R₃ und R₄ zusammen für eine Oxogruppe,
oder bilden R₃ und R₄ unter Einschluß des C-17 ein Spirooxiran oder ein 2,2-Dimethyl-1,3-dioxolan
und der 14,15-Cyclopropanring entweder α- oder β-ständig angeordnet ist, wobei R₂ α-ständig ist, wenn der Cyclopropanring β-ständig ist und umgekehrt.

Die erfindungsgemäßen Verbindungen, die neuen 14,15-Cyclopropanosteroide der 19-Norandrostan-Reihe, sind bisher nicht beschrieben. Ihre biologische Wirkung ist noch nicht bekannt.

Aufgabe der vorliegenden Erfindung ist es, 14,15-Cyclopropanosteroide der 19-Norandrostan-Reihe der allgemeinen Formel 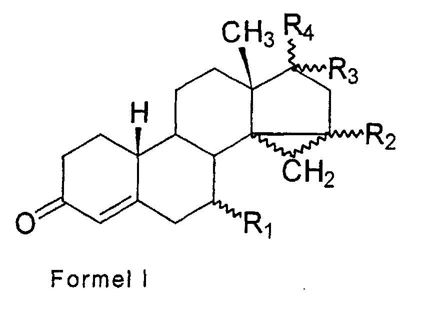 und ihre pharmazeutisch annehmbaren Salze sowie ein Verfahren zu ihrer Herstellung zur Verfügung zu stellen.
Eine weitere Aufgabe ist es, pharmazeutische Präparate, die mindestens eine Verbindung der allgemeinen Formel I oder deren pharmazeutisch annehmbaren Salze enthalten, zur Verfügung zu stellen.

In der allgemeinen Formel I 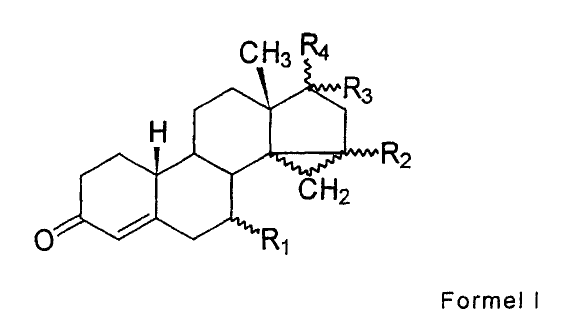
ist R₁ ein Wasserstoffatom oder ein Alkylrest mit 1-9 Kohlenstoffatomen,
steht R₂ für ein Wasserstoffatom oder eine Methylgruppe,
stehen R₃ und R₄ unabhängig voneinander für ein Wasserstoffatom,
für eine Hydroxygruppe, für eine Acyloxygruppe -O-CO-R₅ mit R₅ für 1-10 Kohfenstoffatome,
für eine Carbamoyloxygruppe -O-CO-NHR6 mit R₆ für ein Wasserstoffatom, einen Alkyl-, Arylrest mit jeweils 1-5 Kohlenstoffatomen,
für eine Sulfamoyloxygruppe -O-SO2-NR₇R₈ mit R₇ und R₈ unabhängig voneinander jeweils für ein Wasserstoffatom, eine Alkylgruppe mit 1-5 Kohlenstoffatomen oder zusammen mit dem Stickstoffatom für eine Pyrrolidino-, Piperidino- oder Morpholinogruppe,
für eine Gruppierung -CH₂R₉ mit R₉ für eine Hydroxygruppe, eine Alkyloxygruppe mit 1-5 C-Atomen, ein Chlor- oder Bromatom, eine Azido-, Nitrilo- oder Thiocyanogruppe oder für eine Gruppierung -SR₁₀ mit R₁₀ für eine Alkylgruppe mit 1-5 Kohlenstoffatomen,
oder stehen R₃ und R₄ zusammen für eine Oxogruppe,
oder bilden R₃ und R₄ unter Einschluß des C-17 ein Spirooxiran oder ein 2,2-Dimethyl-1,3-dioxolan
und der 14,15-Cyclopropanring entweder α- oder β-ständig angeordnet ist, wobei R₂ α-ständig ist, wenn der Cyclopropanring β-ständig ist und umgekehrt.

Am meisten bevorzugt sind
17β-Hydroxy-14α,15α-methylenestr-4-en-3-on (J 1129),
17α-Hydroxy-14α,15α-methylenestr-4-en-3-on,
17β-Hydroxy-15β-methyl-14α,15α-methylenestr-4-en-3-on,
17β-Hydroxy-15α-methyl-14β,15β-methylenestr-4-en-3-on,
17β-Hydroxy-17α-hydroxymethyl-14α,15α-methylenestr-4-en-3-on,
17α-Methoxy-17β-methyloxymethyl-14α,15α-methylenestr-4-en-3-on,
17β-Hydroxy-7α-methyl-14α,15α-methylenestr-4-en-3-on,
17,20-Isopropylidendioxy-14α,15α-methylen-19,21-bisnor-17α-pregn-4-en-3-on,
3-Oxo-14α,15α-methylenestr-4-en-17β-yl-sulfamat,
3-Oxo-14α,15α-methylenestr-4-en-17β-yl-*n*-butanoat,
17β-Hydroxy-17α-methyloxymethyl-14β,15β-methylenestr-4-en-3-on (J 1222),
17α-Ethylthiomethyl-17β-hydroxy-14β,15β-methylenestr-4-en-3-on, (J1411)
17α-Chlormethyl-17β-hydroxy-14β,15β-methylenestr-4-en-3-on (J 1364),
17α-Azidomethyl-17β-hydroxy-14β,15β-methylenestr-4-en-3-on, (J 1370)
17α-Brommethyl-17β-hydroxy-14β,15β-methylenestr-4-en-3-on (J 1424),
17β-Hydroxy-17α-rhodanomethyl-14β,15β-methylenestr-4-en-3-on (J 1470)
17α-Cyanomethyl-17β-hydroxy-14β,15β-methylenestr-4-en-3-on (J 1517)

Die Erfindung betrifft ferner ein Verfahren zur Herstellung der Verbindungen nach der allgemeinen Formel und deren pharmazeutisch annehmbaren Salzen, das dadurch gekennzeichnet ist, daß man eine Verbindung der allgemeinen Formel II, 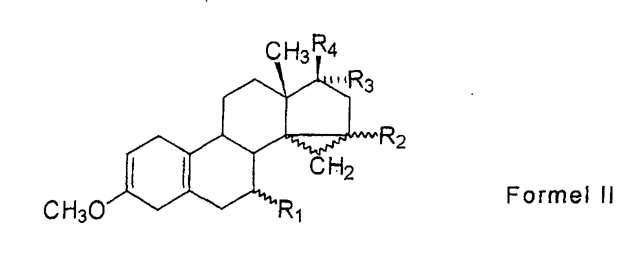 worin R₁, R₂, R₃ und R₄ die vorstehend angegebene Bedeutung besitzt in an sich bekannter Weise durch Enoletherspaltung herstellt.
Die Enoletherspaltung wird durch Einwirkung starker Säuren, wie Schwefelsäure, Salzsäure oder organische Sulfonsäuren auf die in einem geeigneten organischen Lösungsmittel gelösten Verbindungen der allgemeinen Formel I durchgeführt.

Gegenstand der vorliegenden Erfindung sind Arzneistoffe zur oralen, rektalen, subcutanen, intravenösen oder intramuskulären Anwendung, die zusammen mit den üblichen Trägestoffen und Verdünnungsmitteln mindestens eine Verbindung der allgemeinen Formel I oder deren Säureadditionssalze als Wirkstoff enthalten.

Pharmazeutische Präparate der Erfindung werden mit den üblichen festen oder flüssigen Trägerstoffen und/oder Verdünnungsmitteln und den allgemein üblicherweise verwendeten Hilfsstoffen entsprechend der gewünschten Applikationsart in einer geeigneten Dosierung und in an sich bekannter Weise hergestellt. Bei einer bevorzugten oralen Darreichungsform werden vorzugsweise Tabletten, Filmtabletten, Dragees, Kapseln, Pillen, Pulver, Lösungen oder Suspensionen auch als Depotform zubereitet.
Daneben sind parenterale Arzneiformen wie Injektionslösungen oder aber Suppositorien in Betracht zu ziehen.
Arzneiformen als Tabletten können beispielsweise durch Mischen des Wirkstoffes mit bekannten Hilfsstoffen, wie Dextrose. Zucker, Sorbit, Mannit, Polyvinylpyrrolidon, Sprengmitteln wie Maisstärke oder Alginsäure Bindemitteln wie Stärke oder Gelatine, Gleitmitteln wie Magnesiumstearat oder Talk und/oder Mitteln, die einen Depoteffekt erzielen können, wie Carboxylpolymethylen, Carboxymethylcellulose, Celluloseacetatphthalat oder Polyvinylacetat, erhalten werden. Die Tabletten können auch aus mehreren Schichten bestehen.
Analog lassen sich Dragees durch Überziehen von analog den Tabletten hergestellten Kernen mit üblicherweise in Drageeüberzügen verwendeten Mitteln, beispielsweise Polyvinylpyrrolidon oder Schellack, Gummiarabicum, Talk, Titandioxid, oder Zucker bereiten. Die Drageehülle kann dabei auch aus mehreren Schichten bestehen, wobei beispielsweise oben genannte Hilfsstoffe verwendet werden.
Die Lösungen oder Suspensionen mit dem erfindungsgemäßen Wirkstoff können zur Verbesserung des Geschmacks mit Stoffen wie Saccharin, Cyclamat oder Zucker und / oder mit Aromastoffen, wie Vanillin oder Orangenextrakt versetzt werden. Weiterhin können sie mit Suspendierhilfsstoffen wie Natriumcarboxymethylcellulose oder Konservierungsmitteln wie p-Hydroxybenzoesäure vermischt werden.
Die Bereitung von Kapseln kann durch Mischen des Arzneistoffes mit Trägern wie Milchzucker oder Sorbit erfolgen, die dann in die Kapseln eingebracht werden.
Die Herstellung von Suppositorien erfolgt vorzugsweise durch Mischung des Wirkstoffes mit geeigneten Trägermaterialien wie Neutralfetten oder Polyethylenglykolen oder dessen Derivaten.
Bei den pharmazeutischen Zubereitungsformen kann es sich weiterhin um perkutane Zubereitungsformen, z.B. Transdermale Therapeutische Systeme (TTS) oder Gele, Sprays oder Salben oder um intranasale Zubereitungsformen wie Nasenspray oder Nasentropfen handeln.

Die erfindungsgemäßen 14,15-Cyclopropanosteroide der 19-Norandrostan-Reihe der allgemeinen Formel I sind hormonell (gestagen und/oder androgen) wirkende Verbindungen.
So ist beispielsweise die Verbindung der allgemeinen Formel I, , in der R₁ und R₂ jeweils ein Wasserstoffatom darstellen, R₃ eine α-ständige Methyloxymethylgruppe und R⁴ eine β-ständige Hydroxygruppe bedeutet sowie die Cyclopropanogruppe β-ständig angeordnet ist,
17β-Hydroxy-17α-methyloxymethyl-14β,15β-methylenestr-4-en-3-on (J 1222), im Schwangerschaftserhaltungstest an der Maus genau so wirksam wie das weltweit zur oralen Kontrazeption eingesetzte Gestagen Norethisteronacetat.
Ein anderes Beispiel ist die Verbindung der allgemeinen Formel I, in der R₁ und R₂ jeweils ein Wasserstoffatom darstellen, R₃ ein α-ständiges Wasserstoffatom bedeutet, R₄ für eine β-ständige Hydroxygruppe steht und der Cyclopropanring α-ständig ist, 17β-Hydroxy-14α,15α-methylenestr-4-en-3-on (J 1129), die im gleichen Test eine etwa 50-fach stärkere gestagene Wirkung aufweist im Vergleich zur Referenzsubstanz Norethisteronacetat.
Während die Substanz 17β-Hydroxy-17α-methyloxymethyl-14β,15β-methylenestr-4-en-3-on (J 1222) mit 52 ± 4 % an den Progesteronrezeptor des Kaninchenuterus bindet (Referenzsubstanz: Progesteron) besteht praktisch keine Affinität zum Androgenrezeptor der Rattenprostata (Referenzsubstanz: 17β-Hydroxy-17α-methyl-estra- 4,9,11-trien-3-on; R 1881).
Hingegen zeigt 17β-Hydroxy-14α,15α-methylenestr-4-en-3-on (J 1129) eine 59 ± 7 %ige Bindung an den Andogenrezeptor, was der Bindungsaffinität des natürlich vorkommenden männlichen Sexualhormons Testosteron entspricht, und eine 42 ± 5 % ige Bindung an den Progesteronrezeptor.
Ein weiteres Beispiel ist die Verbindung der allgemeinen Formel I, in der R₁ und R₂ jeweils ein Wasserstoffatom darstellen, R₃ eine α-ständige Chlormethylgruppe bedeutet, R4 für eine β-ständige Hydroxygruppe steht und der Cyclopropanring β-ständig ist, 17α-Chlormethyl-17β-hydroxy-14β,15β-methylenestr-4-en-3-on (Code J 1364), die mit 710 ± 80 % an den Progesteronrezeptor des Kaninchenuterus bindet (Referenzsubstanz: Progesteron).
Diese Testergebnisse eröffnen den erfindungsgemäßen Verbindungen der allgemeinen Formel I vielfältige Möglichkeiten für die Fertilitätskontrolle bei Mann und Frau, die Hormonreplacement-Therapie bei Mann und Frau oder die Behandlung hormonell bedingter Erkrankungen bei Mann und Frau , wie beispielsweise Endometriose, Mammacarcinom oder Hypogonadismus.

Die erfindungsgemäßen Verbindungen der allgemeinen Formel I sollen an den nachfolgenden Beispielen näher erläutert, jedoch nicht eingeschränkt werden.

### Beispiel 1

### 17α-Ethylthiomethyl-17β-hydroxy-14β,15β-methylenestr-4-en-3-on

500 mg *17(S)*-3-Methoxy-17-spiroepoxy-14β,15β-methylenestra-2,5(10)-dien werden in 10 ml DMSO mit 270 mg Natriumethylthiolat 4 h auf 80 °C erwärmt. Man gießt in wäßrige Natriumchlorid-Lösung ein, saugt den braunen Niederschlag ab und wäscht neutral. Man löst mit Aceton von der Fritte und engt die Lösung unter Vakuum ein. Es verbleibt ein braunes Öl, das durch präparative Schichtchromatographie an Kieselgel PF₂₅₄₊₃₆₆ₙₘ (MERCK AG) mit dem Laufmittelgemisch Toluol/Aceton 50 :1 gereinigt wird.
225 mg 17α-Ethylthiomethyl-3-methoxy-17β-hydroxy-14β,15β-methylenestra-2,5(10)-dien werden in 3,0 ml Dimethylformamid mit 0,3 ml HCl (konz.) innerhalb von 1 h umgesetzt. Nach Zugabe von wäßriger NaHCO₃-Lösung wird neutralisiert, mit CH₂Cl₂ extrahiert, die organische Phase neutral gewaschen , über Natriumsulfat getrocknet und unter Vakuum verdampft. Man erhält ein Öl, das durch präparative Schichtchromatographie an Kieselgel PF₂₅₄₊₃₆₆ₙₘ (MERCK AG) mit dem Laufmittelgemisch Toluol/Aceton 10 :1 gereinigt wird.
Nach der Umkristallisation aus Ethanol wird 17α-Ethylthiomethyl-17β-hydroxy-14β,15β-methylenestr-4-en-3-on erhalten.
Fp.: 92 -98°C; ¹H-NMR (CDCl₃/TMS): 0,47 (2H, m, 14β, 15β-CH₂), 1,07 (3H, s, H₁₈); 1,28 (3 H, t, SCH₂CH₃), 2,52(2H, q, 14,7 Hz, 7,5 Hz, SCH₂), 2,62 und 2,78 (2H, 2d, 12,6 Hz, 17α CH₂S-); 2,89 (1H, s, OH), 5,81 (1H, s, H₄); *nach TAI:* 0,55 (2H, m, 14β, 15β-CH₂), 1,21 (3 H, t, SCH₂CH₃), 1.34 (3H, s, H₁₈); 2,46 (2H, q, 7,5 Hz, 14,7 Hz, SCH₂), 2,82 und 3,00 (2H, 2d, 13,2 Hz, 17α CH₂S-); 5,81 (1H, s, H₄); 8,21 (s, NHCO) ppm

### Beispiel 2

### 17α-Chlormethyl-17β-hydroxy-14β,15β-methylenestr-4-en-3-on (J 1364)

625 mg *17(S)*-3-Methoxy-17-spiroepoxy-14β,15β-methylenestra-2,5(10)-dien werden in 10 ml DMF gelöst und mit 1ml HCI (konz.) bei Raumtemperatur versetzt. Man rührt 1,5 h, gießt in Eiswasser ein, saugt den ausgefallenen Niederschlag ab, wäscht neutral und trocknet an der Luft. Das Rohprodukt wird durch präparative Schichtchromatographie an Kieselgel PF₂₅₄₊₃₆₆ₙₘ (MERCK AG) mit dem Laufmittelgemisch Toluol/Aceton 15 :1 gereinigt.
Nach der Umkristallisation aus Aceton erhält man 17α-Chlormethyl-17β-hydroxy-14β,15β-methylenestr-4-en-3-on.
Fp.: 177-179 °C; α_{D} = +67° (CHCl₃), ¹H-NMR (CDCl₃/TMS + TAI): 0,48(2H, m, 14β,15β-CH₂), 1,34 (3H, s, H₁₈); 2,15 (1H, s, OH), 3,56 und 3,67 (2H, 2d, 8,4 Hz,
17α-CH₂Cl); 5,79 (1H, s, H₄) ppm.

### Beispiel 3

### 17α-Azidomethyl-17β-hydroxy-14β,15β-methylenestr-4-en-3-on

550 mg *17(S)*-3-Methoxy-17-spiroepoxy-14β,15β-methylenestra-2,5(10)-dien werden in 35 ml Ethylenglykol mit 2,6 g Natriumazid 6 h auf 100 °C erwärmt. Man gießt in Eiswasser ein, extrahiert mit CH₂Cl₂, wäscht neutral, trocknet über Natriumsulfat und engt die Lösung unter Vakuum ein. Das Rohprodukt wird durch präparative Schichtchromatographie an Kieselgel PF₂₅₄₊₃₆₆ₙₘ (MERCK AG) mit dem Laufmittelgemisch Toluol/Aceton 50 :1 gereinigt. Nach Umkristallisation aus Aceton erhält man 17α-Azidomethyl-17β-hydroxy-14β,15β-methylenestra2,5(10)-3-methylether als Kristalle vom Schmelzpunkt 138-143 °C erhalten.
285 mg 17α-Azidomethyl-17β-hydroxy-3-methoxy-14β,15β-methylenestra-2,5(10)-dien werden in 4 ml Dimethylformamid mit 0,4 ml HCI (konz.) innerhalb von 2 h umgesetzt. Nach Zugabe von wäßriger NaHCO₃-Lösung wird neutralisiert, mit CH₂Cl₂ extrahiert, die organische Phase neutral gewaschen , über Natriumsulfat getrocknet und unter Vakuum verdampft. Man erhält ein gelbes Öl, das durch präparative Schichtchromatographie an Kieselgel PF₂₅₄₊₃₆₆ₙₘ (MERCK AG) mit dem Laufmittelgemisch Toluol/Aceton 20 :1 gereinigt wird.
Nach der Umkristallisation aus tert. Butylmethylether /Hexan wird 17α-Azidomethyl-17β-hydroxy-14β,15β-methylenestr-4-en-3-on erhalten.
Fp.: 160-163 °C; α_{D} = +84° (CHCl₃), ¹H-NMR (CDCl₃/TMS): 0,54(2H, m, 14β,15β-CH₂), 1,28 (3H, s, H₁₈); 2,03 (1H, s, OH), 3,34 und 3,46 (2H, 2d, 12,0 Hz, CH₂N₃) 5,81 (s, 1H, H₄) ppm.

### Beispiel 4

### 17β-Hydroxy-17a-(methoxymethyl)-14β,15β-methylenestr-4-en-3-on (J 1222)

3,42 g 17α-(Methoxymethyl)-3-methoxy-14β,15β-methylenestra-2,5(10)-dien-17β-ol werden in 70 ml MeOH suspendiert und mit 3,4 ml HCI (konz.) bei Raumtemperatur versetzt. Man rührt 2 h, versetzt mit 50 ml Wasser und neutralisiert mit wässriger Bicarbonat-Lösung. Der ausgefallene Niederschlag wird abgesaugt, neutral gewaschen und an der Luft getrocknet. Ausbeute: 3,19 g Rohprodukt. Reinigung durch Säulenchromatographie an Kieselgel (0,063-0,2 mm MERCK AG) mit einem Toluol/Aceton Gradienten.
Nach der Umkristallisation aus Isopropanol werden 2,4 g 17β-Hydroxy-17α-(methoxymethyl)-14β,15β-methylenestr-4-en-3-on erhalten.
Fp.: 113-115 °C (Isopropanol); α_{D} = = + 49° (CHCl₃), ¹H-NMR (CDCl₃/TMS): 0,47 (2H, m, 14β,15β-CH₂), 1,10 (3H, s, H₁₈); 2,71 (1H, s, OH), 3,17 und 3,44 (2H, 2d, 9,0 Hz, 17α-*CH*₂OCH₃); 3,33 (1H, s, OCH₃), 5,81 (1H, s, H₄) ppm.

### Beispiel 5

### 17β-Hydroxy-14α,15α-methylenestr-4-en-3-on (J 1129)

Eine Lösung aus 2g 17β-Hydroxy-14α,15α-methylenestr-5-en-3-on in 95 ml Aceton und 5 ml 5%iger wäßriger Chlorwasserstoffsäure wird 2,5 h bei Raumtemperatur gerührt. Anschließend gibt man nacheinander 10 ml gesättigte wäßrige Natriumhydrogencarbonatlösung und 200 ml Wasser hinzu. Diese Lösung wird im Vakuumrotationsverdampfer eingeengt, wobei Kristallisation einsetzt. Die Kristalle werden abfiltriert, getrocknet, in Aceton gelöst und auf eine Kieselgelsäule (0,063 -0,2 mm, Merck) aufgetragen. Chromatographie mit Cyclohexan-Ethylacetat 7:3 (v:v) als Eluent ergibt 17β-Hydroxy-14α,15α-methylenestr-4-en-3-on.
Fp. 168-170 °C; [α]_{D}²⁰ +67° (Pyridin); ¹H-NMR (CDCl₃/TMS + TAI): 0,265 (1H, dd, 5,8 Hz, 3,2 Hz, 14α,15α-CH₂); 0,338 (1H, t, 7 Hz, 14α,15α-CH₂); 1,13 (3H, s, H₁₈); 4,58 (1H, dd, H_{17α}); 5,82 ( s, H₄); 8,41 (s, NH) ppm.

### Beispiel 6

### 17α-Hydroxy-14α,15α-methylenestr-4-en-3-on

17α-Hydroxy-14α,15α-methylenestr-5-en-3-on wird analog Beispiel 5 mit Chlorwasserstoffsäure in Aceton behandelt und das Produkt einer Chromatographie unterworfen, wobei man 17α-Hydroxy-14α,15α-methylenestr-4-en-3-on erhält.
¹H-NMR (CDCl₃/TMS + TAI): 0,39 (1H, dd, 7,2 Hz, 4,9 Hz, 14α,15α-CH₂); 0,67(1H, dd, 4,9 Hz, 2,8 Hz, 14α,15α-CH₂); 1,12 (3H, s, H₁₈); (4,96, 1H, d, 6,1 Hz, H_{17β}); 5,81
(s, H₄); 8,42 (s, NH) ppm.

### Beispiel 7

### 17β-Hydroxy-17α-hydroxymethyl-14α,15α-methylenestr-4-en-3-on

17β-Hydroxy-17α-hydroxymethyl -14α,15α-methylenestr-5-en-3-on wird analog Beispiel 5 mit Chlorwasserstoffsäure in Aceton behandelt und das Produkt einer Chromatographie unterworfen, wobei man 17β-Hydroxy-17α-hydroxymethyl -14α,15α-methylenestr-4-en-3-on erhält.
¹H-NMR (CDCl₃/TMS + TAI): 0,26 (1H, dd, 6,8 Hz, 2,0 Hz, 14α,15α-CH₂); 0,51 (1H, t, 6,8 Hz, 14α,15α-CH₂); 1,26 (3H, s, H₁₈); 4,58, 4,93 (2H, AB, 12,2 Hz, CH₂O); 5,82 (1H, t, 2,2 Hz, H₄); 8,44 (1H, s, NH); 8,52 (1H, s, NH) ppm.

### Beispiel 8

### 17α-Methoxy-17β-methoxymethyl-14α,15α-methylenestr-4-en-3-on

17α-Methoxy-17β-methoxymethyl-14α,15α-methylenestr-5-en-3-on wird analog Beispiel 5 mit Chlorwasserstoffsäure in Aceton behandelt und das Produkt einer Chromatographie unterworfen, wobei man 17α-Methoxy-17β-methoxymethyl-14α,15α-methylenestr-4-en-3-on erhält.
¹H-NMR (CDCl₃/TMS): 0,18 (1H, m, 14α,15α-CH₂); 0,954 (1H, t, 3,3 Hz, 14α,15α-CH₂); 1,11 (3H, s, H₁₈); 3,15 (3H, s, OCH₃); 3,31 (3H, s, OCH₃); 3,26, 3,52 (2H, AB, 11,2 Hz, CH₂O), 5,80 (1H, t, 2 Hz, H₄) ppm.

### Beispiel 9

### 17,20-Isopropylidendioxy-14α,15α-methylen-19,21-bisnor-17α-preg n-4-en-3-on

17,20-Isopropylidendioxy-14α,15α-methylen-19,21-bisnor-17α-pregn-5-en-3-on wird analog Beispiel 5 mit Chlorwasserstoffsäure in Aceton behandelt und das Produkt einer Chromatographie unterworfen, wobei man 17,20-lsopropylidendioxy-14α,15α-methylen-19,21-bisnor-17α-pregn-4-en-3-on erhält.
¹H-NMR (CDCl₃/TMS): -0,27 (1H, m, 14α,15α-CH₂); 0,32 (t, 14α,15α-CH₂); 1,14 (3H, s, H₁₈); 1,30 (3H, s, OCH₃); 1,37 (3H, s, OCH₃); 3,50, 4,07 (2H, AB, 8,5 Hz, CH₂O), 5,81 (1H, t, 2,2 Hz, H₄) ppm.

### Beispiel 10

### 17α-Brommethyl -17β-hydroxy-14β,15β-methylenestr -4-en-3-on

666 mg 17(S)-Spiroepoxy-14β,15β-methylenestra-2,5(10)-dien-3-methylether wer-den in 10 ml DMF gelöst und mit 2,5 ml HBr (48 %) bei Raumtemperatur versetzt. Man rührt 4 h, gießt in Eiswasser ein, saugt den ausgefallenen Niederschlag ab, wäscht neutral und trocknet an der Luft. Das Rohprodukt wird durch präparative Schichtchromatographie an Kieselgel PF₂₅₄₊₃₆₆ₙₘ (MERCK AG) mit dem Laufmittelgemisch Toluol/Aceton 20:1 gereinigt.
Nach zweimaliger Umkristallisation aus Aceton werden 324 mg 17α-Brommethyl-17β-hydroxy-14β,15β-methylenestr-4-en-3-on erhalten.
Fp.: 143 (Zers.) °C; α_{D} = +68° (CHCl₃), ¹H-NMR (CDCl₃, ppm, TMS): 0,50 (m, 2H, 14β,15β-CH₂), 1,15 (s, 3H, H-18); 2,27 (s, 1H, OH), 3,57 (t, 2H, J= 10,2 Hz, H-17α-CH₂Br); 5,81 (s, 1H, H-4);

### Beispiel 11

### 17β-Hydroxy-17α-rhodanomethyl-14β,15β-methylenestr -4-en-3-on

666 mg 17(S)-Spiroepoxy-14β,15β-methylenestra-2,5(10)-dien-3-methylether werden in 10 ml DMF gelöst und mit 2,5 ml HBr (48 %) bei Raumtemperatur versetzt. Man rührt 4 h, gießt in Eiswasser ein, saugt den ausgefallenen Niederschlag ab, wäscht neutral und trocknet an der Luft. Das Rohprodukt wird durch präparative Schichtchromatographie an Kieselgel PF₂₅₄₊₃₆₆ₙₘ (MERCK AG) mit dem Laufmittelgemisch Toluol/Aceton 20:1 gereinigt.
Nach dreimaliger Umkristallisation aus Essigester und MeOH werden 185 mg 17β-Hydroxy-17α-rhodanomethyl-14β,15β-methylenestr-4-en-3-on erhalten.
Fp.: 168-173 °C (MeOH); α_{D} = +86° (CHCl₃), ¹H-NMR (CDCl₃, ppm,TMS): 0,53 (m, 2H, 14β,15β-CH₂), 1,11 (s, 3H, H-18); 1,98 (s, 1H, OH), 3,21 (t, 2H, J= 10,2 Hz, H-17α-CH₂SCN); 5,82 (s, 1H, H-4).

### Beispiel 12

### 17α-Cyanomethyl -17β-hydroxy-14β,15β-methylenestr -4-en-3-on

624 mg 17(S)-Spiroepoxy-14β,15β-methylenestra-2,5(10)-dien-3-methylether wer-den in 15 ml Ethanol suspendiert und mit 490 mg Natriumcyanid bei Raumtemperatur bis zum vollständigen Umsatz gerührt. Anschließend gießt man in Eiswasser ein, saugt ab, wäscht neutral und trocknet an der Luft. Das Rohprodukt von 17α-Cyanomethyl-3-methoxy-14β,15β-methylenestra-2,5(10)-dien-17β-ol wird durch präparative Schichtchromatographie an Kieselgel PF₂₅₄₊₃₆₆ₙₘ (MERCK AG) mit dem Laufmittelgemisch Toluol/Aceton 20:1 gereinigt, in 10 ml DMF gelöst und mit 0,5 ml HCI versetzt. Nach 2 Stunden wird in Eiswasser eingegossen, wird der Niederschlag abgesaugt und durch Chromatographie gereinigt. Die Umkristallisation von 17α-Cyanomethyl-17β-hydroxy-14β,15β-methylenestr-4-en-3-on erfolgt aus Aceton.

Fp.: 244-253 °C; α_{D} = + 65° (CHCl₃), ¹H-NMR (CDCl₃/TMS): 0,56 (m, 2H, 14β,15β-CH₂), 1,17 (s, 3H, H-18); 2,28 (s, 1H, OH), 2,3-2,5 (m, H-17α-CH₂CN); 5,82 (s, 1H, H-4);

## Patentansprüche

1. 14,15-Cyclopropanosteroide der 19-Norandrostan-Reihe der allgemeinen Formel I 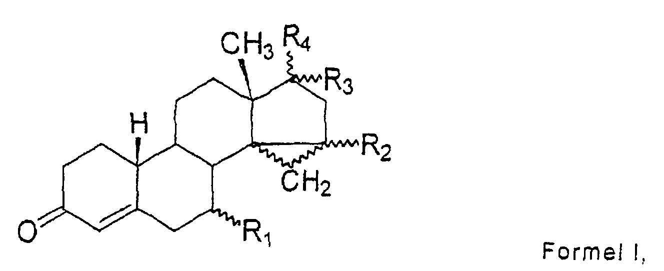 worin
R₁ ein Wasserstoffatom oder eine Alkylrest mit 1-9 Kohlenstoffatomen ist,
R₂ für ein Wasserstoffatom oder eine Methylgruppe steht
R₃ und R₄ unabhängig voneinander für ein Wasserstoffatom,
für eine Hydroxygruppe,
für eine Acyloxygruppe -O-CO-R₅
mit R₅ für 1-10 Kohlenstoffatome,
für eine Carbamoyloxygruppe -O-CO-NHR₆
mit R₆ für ein Wasserstoffatom, einen Alkyl-, Arylrest mit jeweils 1-5 Kohlenstoffatomen,
für eine Sulfamoyloxygruppe -O-SO₂-NR₇R₈,
mit R₇ und R₈ unabhängig voneinander für jeweils ein Wasserstoffatom, eine Alkylgruppe
mit 1- 5 Kohlenstoffatomen oder zusammen mit dem Stickstoffatom für eine Pyrrolidino-, Piperidino- oder Morpholinogruppe,
für eine Gruppierung -CH₂R₉ mit R₉ für eine Hydroxygruppe, eine Alkyloxygruppe mit 1-5 Kohlenstoffatomen, ein Chlor- oder Bromatom, eine Azido-, Nitrilo- oder Thiocyanogruppe oder
für eine Gruppierung -SR₁₀ steht mit R₁₀ für eine Alkylgruppe mit 1-5 Kohlenstoffatomen
stehen,
R₃ und R₄ zusammen für eine Oxogruppe stehen,
R₃ und R₄ unter Einschluß des C-17 ein Spirooxiran oder ein 2,2-Dimethyl-1,3-dioxolan bilden.

2. Verbindungen nach Anspruch 1, nämlich
17β-Hydroxy-14α,15α-methylenestr-4-en-3-on (J 1129),
17α-Hydroxy-14α,15α-methylenestr-4-en-3-on,
17β-Hydroxy-15β-methyl-14α,15α-methylenestr-4-en-3-on,
17β-Hydroxy-15α-methyl-14β,15β-methylenestr-4-en-3-on,
17β-Hydroxy-17α-hydroxymethyl-14α,15α-methylenestr-4-en-3-on,
17α-Methoxy-17β-methyloxymethyl-14α,15α-methylenestr-4-en-3-on,
17β-Hydroxy-7α-methyl-14α,15α-methylenestr-4-en-3-on,
17,20-Isopropylidendioxy-14α,15α-methylen-19,21-bisnor-17α-pregn-4-en-3-on,
3-Oxo-14α,15α-methyfenestr-4-en-17β-yl-sulfamat;
3-Oxo-14α,15α-methylenestr-4-en-17β-yl-*n*-butanoat,
17β-Hydroxy-17α-methyloxymethyl-14β,15β-methylenestr-4-en-3-on (J 1222),
17α-Ethylthiomethyl-17β-hydroxy-14β,15β-methylenestr-4-en-3-on (J 1411),
17α-Chlormethyl-17β-hydroxy-14β,15β-methylenestr-4-en-3-on (J 1364).
17α-Azidomethyl-17β-hydroxy-14β,15β-methylenestr-4-en-3-on (J 1370),
17α-Brommethyl-17β-hydroxy-14β,15β-methylenestr-4-en-3-on (J 1424),
17β-Hydroxy-17α-rhodanomethyl-14β,15β-methylenestr-4-en-3-on (J 1470),
17α-Cyanomethyl-17β-hydroxy-14β,15β-methylenestr-4-en-3-on (J 1517)

3. Verfahren zur Herstellung der Verbindungen nach Anspruch 1 und deren pharmazeutisch annehmbaren Salzen,
**dadurch gekennzeichnet,**
**daß** man 14,15-Cyclopropano-enolether der allgemeinen Formel II, 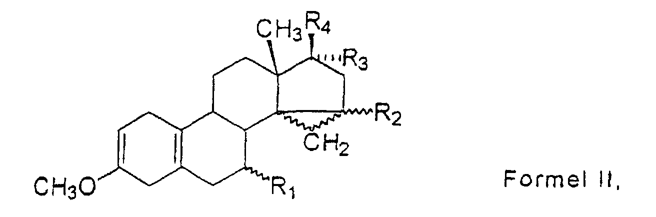 worin
R₁ ein Wasserstoffatom oder eine Alkylrest mit 1-9 Kohlenstoffatomen ist,
R₂ für ein Wasserstoffatom oder eine Methylgruppe steht,
R₃ und R₄ unabhängig voneinander für ein Wasserstoffatom,
für eine Hydroxygruppe,
für eine Acyloxygruppe -O-CO-R₅
mit R₅ für 1-10 Kohlenstoffatome,
für eine Carbamoyloxygruppe -O-CO-NHR₆ mit R₆ für ein Wasserstoffatom, einen Alkyl-, Arylrest mit jeweils 1-5 Kohlenstoffatomen,
für eine Sulfamoyloxygruppe -O-SO₂-NR₇R₈,
mit R₇ und R₈ unabhängig voneinander für jeweils ein Wasserstoffatom, eine Alkylgruppe
mit 1- 5 Kohlenstoffatomen oder zusammen mit dem Stickstoffatom für eine Pyrrolidino-, Piperidino- oder Morpholinogruppe,
für eine Gruppierung -CH₂R₉ mit R₉ für eine Hydroxygruppe, eine Alkyloxygruppe mit 1-5 Kohlenstoffatomen, ein Chlor- oder Bromatom, eine Azido-, Nitrilo- oder Thiocyanogruppe oder
für eine Gruppierung -SR₁₀ steht mit R₁₀ für eine Alkylgruppe mit 1-5 Kohlenstoffatomen
stehen,
R₃ und R₄ zusammen für eine Oxogruppe stehen,
R₃ und R₄ unter Einschluß des C-17 ein Spirooxiran oder ein 2,2-Dimethyl-1,3-dioxolan bilden,
in an sich bekannter Weise durch Enoletherspaltung herstellt.

4. Pharmazeutische Zusammensetzung, die mindestens eine Verbindung der allgemeinen Formel (I) nach einem der Ansprüche 1 und 2 oder deren Säureadditionssalz, zusammen mit Verdünnungsmitteln und/oder Trägerstoffen enthält.

5. Verwendung der Verbindungen der allgemeinen Formel (I) nach einem der Ansprüche 1 und 2, zur Herstellung eines Arzneimittels zur Fertilitätskontrolle, der Hormon-Replacement-Therapie (HRT) oder der Behandlung hormonell bedingter Erkrankungen, wie beispielsweise Endometriose, Mammacarcinom oder Hypogonadismus.

6. Verbindungen der allgemeinen Formel (I) nach einem der Ansprüche 1 und 2 zur Anwendung als therapeutische Wirkstoffe.

## Claims

1. 14,15-cyclopropanosteroids of the 19-norandrostane series of the general formula I 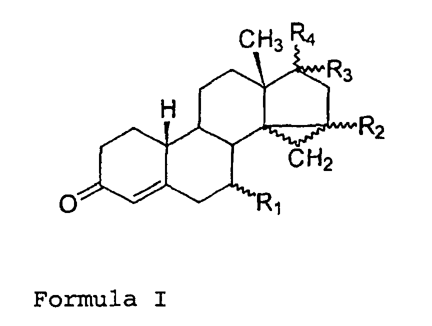 in which
R₁ is a hydrogen atom or an alkyl radical having 1-9 carbon atoms,
R₂ is a hydrogen atom or a methyl group,
R₃ and R₄ independently of one another are a hydrogen atom,
a hydroxyl group,
an acyloxy group -O-CO-R₅
where R₅ is 1-10 carbon atoms,
a carbamoyloxy group -O-CO-NHR₆
where R₆ is a hydrogen atom, an alkyl or aryl radical in each case having 1-5 carbon atoms,
a sulphamoyloxy group -O-SO₂-NR₇R₈
where R₇ and R₈ independently of one another are in each case a hydrogen atom, an alkyl group having 1-5 carbon atoms or together with the nitrogen atom are a pyrrolidino, piperidino or morpholino group,
a group -CH₂R₉ where R₉ is a hydroxyl group, an alkyloxy group having 1-5 carbon atoms, a chlorine or bromine atom,
an azido, nitrilo or thiocyano group or
a group -SR₁₀ where R₁₀ is an alkyl group having 1-5 carbon atoms,
R₃ and R₄ together are an oxo group,
R₃ and R₄, with inclusion of C-17, form a spirooxirane or a 2,2-dimethyl-1,3-dioxolane.

2. Compounds according to Claim 1,
namely
17β-hydroxy-14α,15α-methyleneestr-4-en-3-one (J 1129),
17α-hydroxy-14α,15α-methyleneestr-4-en-3-one,
17β-hydroxy-15β-methyl-14α,15α-methyleneestr-4-en-3-one,
17β-hydroxy-15α-methyl-14β,15β-methyleneestr-4-en-3-one,
17β-hydroxy-17α-hydroxymethyl-14α,15α-methyleneestr-4-en-3-one,
17α-methoxy-17β-methyloxymethyl-14α,15α-methyleneestr-4-en-3-one,
17β-hydroxy-7α-methyl-14α,15α-methyleneestr-4-en-3-one,
17,20-isopropylidenedioxy-14α,15α-methylene-19,21-bisnor-17α-pregn-4-en-3-one,
3-oxo-14α,15α-methyleneestr-4-en-17β-yl sulphamate,
3-oxo-14α,15α-methyleneestr-4-en-17β-yl *n*-butanoate,
17β-hydroxy-17α-methyloxymethyl-14β,15β-methyleneestr-4-en-3-one (J 1222),
17α-ethylthiomethyl-17β-hydroxy-14β,15β-methyleneestr-4-en-3-one (J 1411),
17α-chloromethyl-17β-hydroxy-14β,15β-methyleneestr-4-en-3-one (J 1364),
17α-azidomethyl-17β-hydroxy-14β,15β-methyleneestr-4-en-3-one (J 1370),
17α-bromomethyl-17β-hydroxy-14β,15β-methyleneestr-4-en-3-one (J 1424),
17β-hydroxy-17α-rhodanomethyl-14β,15β-methyleneestr-4-en-3-one (J 1470),
17α-cyanomethyl-17β-hydroxy-14β,15β-methyleneestr-4-en-3-one (J 1517).

3. Process for the preparation of the compounds according to Claim 1 and their pharmaceutically acceptable salts, **characterized in that** 14,15-cyclopropano enol ethers of the general formula II 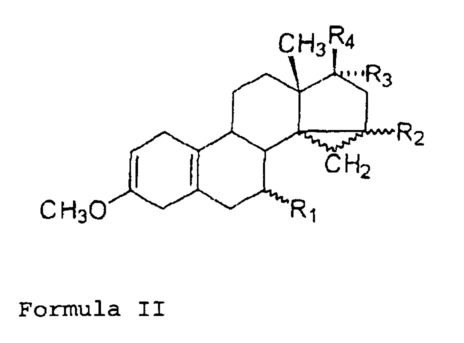 in which
R₁ is a hydrogen atom or an alkyl radical having 1-9 carbon atoms,
R₂ is a hydrogen atom or a methyl group,
R₃ and R₄ independently of one another are a hydrogen atom,
a hydroxyl group,
an acyloxy group -O-CO-R₅
where R₅ is 1-10 carbon atoms,
a carbamoyloxy group -O-CO-NHR₆
where R₆ is a hydrogen atom, an alkyl or aryl radical in each case having 1-5 carbon atoms,
a sulphamoyloxy group -O-SO₂-NR₇R₈
where R₇ and R₈ independently of one another are in each case a hydrogen atom, an alkyl group having 1-5 carbon atoms or together with the nitrogen atom are a pyrrolidino, piperidino or morpholino group,
a group -CH₂R₉ where R₉ is a hydroxyl group, an alkyloxy group having 1-5 carbon atoms, a chlorine or bromine atom,
an azido, nitrilo or thiocyano group or
a group -SR₁₀ where R₁₀ is an alkyl group having 1-5 carbon atoms,
R₃ and R₄ together are an oxo group,
R₃ and R₄, with inclusion of C-17, form a spirooxirane or a 2,2-dimethyl-1,3-dioxolane, are prepared in a manner known per se by enol ether cleavage.

4. Pharmaceutical composition which contains at least one compound of the general formula (I) according to either of Claims 1 and 2 or its acid addition salt, together with diluents and/or vehicles.

5. Use of the compounds of the general formula (I) according to either of Claims 1 and 2 for the production of a medicament for fertility control, hormone replacement therapy (HRT) or the treatment of hormonally related diseases, such as, for example, endometriosis, breast carcinoma or hypogonadism.

6. Compounds of the general formula (I) according to either of Claims 1 and 2 for use as therapeutic active compounds.

## Revendications

1. 14,15-cyclopropanostéroïdes de la série des 19-norandrostanes, de formule générale I 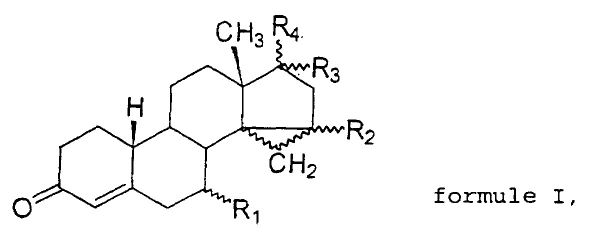 dans laquelle
R₁ représente un atome d'hydrogène ou un radical alkyle ayant de 1 à 9 atomes de carbone,
R₂ représente un atome d'hydrogène ou le groupe méthyle,
R₃ et R₄ représentent, indépendamment l'un de l'autre,
un atome d'hydrogène,
un groupe acyloxy -O-CO-R₅,
R₅ représentant de 1 à 10 atomes de carbone,
un groupe carbamoyloxy -O-CO-NHR₆,
R₆ représentant un atome d'hydrogène, un radical alkyle, aryle, ayant chacun de 1 à 5 atomes de carbone,
un groupe sulfamoyloxy -O-SO₂-NR₇R₈,
R₇ et R₈ représentant chacun, indépendamment l'un de l'autre, un atome d'hydrogène, un groupe alkyle ayant de 1 à 5 atomes de carbone, ou formant ensemble, avec l'atome d'azote, un groupe pyrrolidino, pipéridino ou morpholino,
un groupement -CH₂R₉, R₉ représentant
un groupe hydroxy, un groupe alkyloxy ayant de 1 à 5 atomes de carbone, un atome de chlore ou de brome, un groupe azido, nitrilo ou thiocyano, ou un groupement -SR₁₀,
R₁₀ représentant un groupe alkyle ayant de 1 à 5 atomes de carbone,
R₃ et R₄ représentent ensemble un groupe oxo,
R₃ et R₄ forment, avec inclusion du C-17, un cycle spiro-oxiranne ou un cycle 2,2-diméthyl-1,3-dioxolanne.

2. Composés selon la revendication 1, à savoir
17β-hydroxy-14α,15α-méthylène-estr-4-én-3-one (J 1129),
17α-hydroxy-14α,15α-méthylène-estr-4-én-3-one,
17β-hydroxy-15β-méthyl-14α,15α-méthylène-estr-4-én-3-one,
17β-hydroxy-15α-méthyl-14β,15β-méthylène-estr-4-én-3-one,
17β-hydroxy-17α-hydroxyméthyl-14α,15α-méthylène-estr-4-én-3-one,
17α-méthoxy-17β-méthyloxyméthyl-14α,15α-méthylène-estr-4-én-3-one,
17β-hydroxy-7α-méthyl-14α,15α-méthylène-estr-4-én-3-one,
17,20-isopropylidènedioxy-14α,15α-méthylène-19,21-bisnor-17α-prégn-4-én-3-one,
sulfamate de 3-oxo-14α,15α-méthylène-estr-4-én-17β-yle,
n-butanoate de 3-oxo-14α,15α-méthylène-estr-4-én-17β-yle,
17β-hydroxy-17α-méthyloxyméthyl-14β,15β-méthylène-estr-4-én-3-one (J 1222),
17α-éthylthiométhyl-17β-hydroxy-14β,15β-méthylène-estr-4-én-3-one (J 1411),
17α-chlorométhyl-17α-hydroxy-14β,15β-méthylène-estr-4-én-3-one (J 1364),
17α-azidométhyl-17β-hydroxy-14β,15β-méthylène-estr-4-én-3-one (J 1370),
17α-bromométhyl-17β-hydroxy-14β,15β-méthylène-estr-4-én-3-one (J 1424),
17β-hydroxy-17α-sulfocyanométhyl-14β,15β-méthylène-estr-4-én-3-one (J 1470),
17α-cyanométhyl-17β-hydroxy-14β,15β-méthylène-estr-4-én-3-one, (J 1517).

3. Procédé pour la préparation des composés selon la revendication 1 et de leurs sels pharmaceutiquement acceptables, **caractérisé en ce qu'**on prépare par coupure d'énoléther, d'une façon connue en soi, des 14,15-cyclopropane-énoléthers de formule générale II 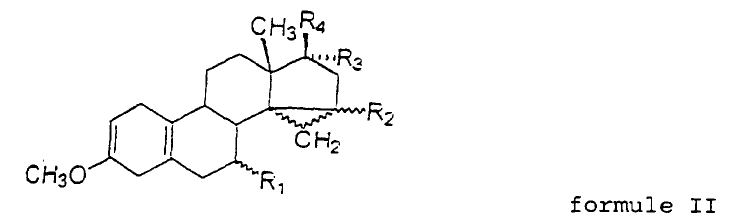 dans laquelle
R₁ représente un atome d'hydrogène ou un radical alkyle ayant de 1 à 9 atomes de carbone,
R₂ représente un atome d'hydrogène ou le groupe méthyle,
R₃ et R₄ représentent, indépendamment l'un de l'autre,
un atome d'hydrogène,
un groupe acyloxy -O-CO-R₅,
R₅ représentant de 1 à 10 atomes de carbone,
un groupe carbamoyloxy -O-CO-NHR₆,
R₆ représentant un atome d'hydrogène, un radical alkyle, aryle, ayant chacun de 1 à 5 atomes de carbone,
un groupe sulfamoyloxy -O-SO₂-NR₇R₈,
R₇ et R₈ représentant chacun, indépendamment l'un de l'autre, un atome d'hydrogène, un groupe alkyle ayant de 1 à 5 atomes de carbone, ou formant ensemble, avec l'atome d'azote, un groupe pyrrolidino, pipéridino ou morpholino,
un groupement -CH₂R₉, R₉ représentant
un groupe hydroxy, un groupe alkyloxy ayant de 1 à 5 atomes de carbone, un atome de chlore ou de brome, un groupe azido, nitrilo ou thiocyano, ou un groupement -SR₁₀
R₁₀ représentant un groupe alkyle ayant de 1 à 5 atomes de carbone,
R₃ et R₄ représentent ensemble un groupe oxo,
R₃ et R₄ forment, avec inclusion du C-17, un cycle spiro-oxiranne ou un cycle 2,2-diméthyl-1,3-dioxolanne.

4. Composition pharmaceutique qui contient au moins un composé de formule générale (I) selon l'une quelconque des revendications 1 et 2 ou un sel d'addition avec un acide de celui-ci, conjointement avec des diluants et/ou véhicules.

5. Utilisation des composés de formule générale (I) selon l'une quelconque des revendications 1 et 2, pour la fabrication d'un médicament destiné à la régulation de la fertilité, à l'hormonothérapie substitutive (HRT) ou au traitement de maladies d'origine hormonale, comme par exemple l'endométriose, le cancer du sein ou l'hypogonadisme.

6. Composés de formule générale (I) selon l'une quelconque des revendications 1 et 2, pour utilisation en tant que substances actives thérapeutiques.
